# EUROPEAN PATENT APPLICATION

(11) **EP 0 700 660 A1**
(43) Date of publication of application: **13.03.1996**
(21) Application number: 95910797.0
(22) Date of filing: 10.03.1995
(51) Int. Cl.: A61B 5/14

(54) **BLOOD SUGAR LEVEL MEASURING APPARATUS**

(30) Priority: 14.03.1994 JP 69019/94
(71) Applicant: SONY CORPORATION, Tokyo 141 (JP); OTAX CO., LTD., Yokohama-shi Kanagawa 223 (JP)
(72) Inventor: TAKASHIMA, Mitsuru, c/o Sony Corporation, Tokyo 141 (JP); KISHIGAMI, Jun, c/o Sony Corporation, Tokyo 141 (JP); SUZUKI, Yoshihiko, Tokyo 164 (JP); OHKURA, Tadahiro, c/o Otax Co., Ltd., Yokohama-shi, Kanagawa 223 (JP)
(74) Representative: Körber, Wolfhart, Dr. rer.nat.
(86) International application number: JP9500408
(87) International publication number: WO9524861

(57) **Abstract**

A blood-sugar level measuring apparatus adapted for irradiating a visible light emitted from a light emitting element into the tissue caused to be placed in blood lost state of a portion of the tissue of the human body to detect, by a light receiving element, a light transmitted through the blood lost tissue or reflected from the blood lost tissue. This blood-sugar level measuring apparatus comprises a light emitting element as described above for irradiating visible light into a portion of the tissue of the living body caused to be placed in blood lost state, and a light receiving element as described above for detecting a light obtained as the result of the fact that visible light is transmitted through the blood lost tissue or reflected from the blood lost tissue to measure absorptivity of visible light irradiated into the blood lost tissue on the basis of a detection output of the light receiving element to computationally determine, by an arithmetic processing unit, a calculated blood-sugar level on the basis of correlation between an absorptivity determined in advance and blood-sugar level and the measured absorptivity. This calculation result is displayed on a display monitor.

## Description

### Technical Field

This invention relates to a blood-sugar level measuring apparatus adapted for measuring blood-sugar level, and more particularly to a blood-sugar level measuring apparatus which permits measurement of blood-sugar level in a simple manner without carrying out gathering of blood.

### Background Art

Hitherto, as a method of measuring blood-sugar level, there has been widely carried out a GOD method utilizing decomposition of glucose by glucose oxidase (GOD). As the GOD method, there are an electrode method of carrying (transferring) electrons produced in reaction of glucose and GOD to the electrode through mediator to measure a current quantity, and so called a test paper method which is colorimetric method utilizing coloration with respect to coloring matter by hydrogen peroxide produced by reaction of glucose and GOD.

Such conventional blood-sugar level measurement method can advantageously measure blood-sugar level in relatively short time, e.g., about 20 seconds to one minute by gathering (collecting) of a very small quantity of blood. However, in order to adopt such blood-sugar level measurement method, it is necessary to carry out gathering of blood using puncture needle. For this reason, in addition to the problem that patient suffers from pain, it is necessary to exchange puncture needles every patients to carry out disinfection. Further, there is the problem of disposal of used puncture needles.

In view of the above, a measurement method capable of measuring blood-sugar level without carrying out blood-gathering using puncture needle has been expected.

In order to attain such object, the inventors of this invention have proposed a blood-sugar level measuring apparatus which can measure blood-sugar level without carrying out blood-gathering using puncture needle as described in the specification and the drawings of the Japanese Patent Application No. 191204/1993. The blood-sugar level measuring apparatus described in the specification and the drawings mentioned above is adapted to irradiate far infrared rays into the tissue of living body (organism) to measure absorptivity when a light of a specific wavelength in the range of wavelength 3200 nm ∼ 3900 nm of the irradiated far infrared rays is transmitted through the tissue to determine a blood-sugar level on the basis of correlation between an absorptivity predetermined in advance and blood-sugar level and the measured absorptivity.

The previously proposed blood-sugar level measuring apparatus by non blood-gathering method carries out measurement of glucose of the entirety of blood in order to measure glucose in blood. Light of a specific wavelength in the range of 3200 nm ∼ 3900 nm of far infrared rays which have been irradiated into the tissue of the living body is absorbed by glucose. In this case, since infrared rays including wavelength of the above-mentioned range are radiated also from the human body, there is the problem that any measurement error might take place by such radiated infrared rays. Moreover, there is the problem that it is not easy to handle infrared rays because such infrared rays cannot be observed by the eye.

Further, since far infrared rays to be irradiated are difficult to be transmitted through the tissue of the human body, it is necessary to allow power of irradiated light to be large. However, such high output far infrared rays irradiating unit is expensive. As a result, it is difficult to cheaply provide a blood-sugar level measuring apparatus. In addition, since when high output far infrared rays are irradiated onto the human body, there is the possibility that the human body tissue might suffer from damage such as burn, etc., there is limitation in output power of far infrared rays. For this reason, it is unable to irradiate far infrared rays of power which are permitted to be transmitted through the tissue of the human body and which give sufficient measurement accuracy, and there is also the possibility that any measurement error may take place.

An object of this invention is to provide a blood-sugar level measuring apparatus which can safely and precisely measure blood-sugar level without causing pain to subject.

Another object of this invention is to provide a blood-sugar level measuring apparatus which can stably measure blood-sugar level in short time by simple configuration.

### Disclosure of the Invention

A blood-sugar level measuring apparatus according to this invention comprises a light emitting element for irradiating visible light with respect to a portion of the tissue of the living body caused to be placed in blood lost state, and a light receiving element for detecting a light obtained as the result of the fact that the visible light has been transmitted through the blood lost tissue or has been reflected from the blood lost tissue. This blood-sugar level measuring apparatus comprises an arithmetic processing unit for measuring absorptivity of visible light irradiated into the blood lost tissue on the basis of a detection output of the light receiving element to calculate a calculated blood-sugar level on the basis of correlation between an absorptivity determined in advance and blood-sugar level and the measured absorptivity. Further, the blood-sugar level measuring apparatus according to this invention comprises a display monitor for displaying calculation result of the arithmetic processing unit.

In the blood-sugar level measuring apparatus, visible light emitted from the light emitting element is irradiated onto the blood lost tissue to detect, by the light receiving element, light which has been transmitted through the blood lost tissue or which has been reflected from the blood lost tissue, whereby measurement of blood-sugar level is carried out.

### Brief Description of the Drawings

FIG. 1 is a block diagram showing an embodiment of a blood-sugar level measuring apparatus according to this invention.

FIG. 2 is a view showing measurement state of blood-sugar level.

FIG. 3 is a characteristic diagram for explaining transmission characteristic of a transmitted light by the blood-sugar level measuring apparatus shown in FIG. 1.

FIG. 4 is a characteristic diagram for explaining correlation between transmitted light and blood-sugar level by the blood-sugar level measuring apparatus shown in FIG. 1.

FIG. 5 is a schematic cross sectional view showing another embodiment of a blood-sugar level measuring apparatus according to this invention.

### Best Mode for Carrying Out the Invention

More practical embodiments of this invention will now be described with reference to the attached drawings.

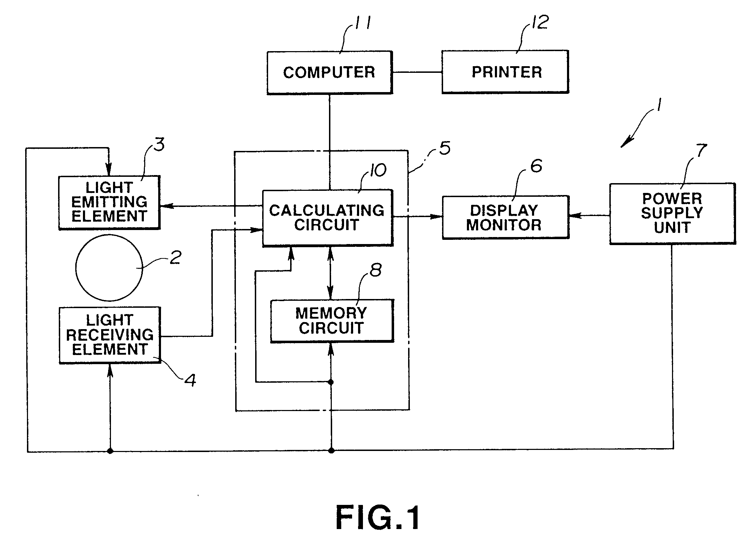

A blood-sugar level measuring apparatus of a first embodiment comprises, as shown in FIG. 1, a light emitting element 3 affixed to a portion 2 of the human body, e.g., finger, etc. and adapted for irradiating visible light into the human body tissue, and a light receiving element 4 adapted to receive light transmitted through the human body tissue to output a light receiving signal corresponding to absorptivity by the human body tissue. In this embodiment, it is desirable to use light of wavelength of 400 nm ∼ 630 nm as visible light. Accordingly, a light emitting element capable of emitting light including light of wavelength of 400 nm ∼ 630 nm is used as the light emitting element 3.

The light emitting element 3 and the light receiving element 4 are disposed so that they are opposite to each other through a portion of the human body tissue, e.g., finger.

Further, the blood-sugar level measuring apparatus 1 comprises an arithmetic processing unit 5 for calculating blood-sugar level on the basis of a signal from the light receiving element 4 and correlation data between an absorptivity determined in advance and blood-sugar level, a display monitor 6 for displaying result of the arithmetic processing unit 5, and a power supply unit 7 for delivering power to these units. In this embodiment, the display monitor 6 is comprised of display element such as liquid crystal panel, etc.

It is to be noted that the arithmetic processing unit 5, the display monitor 6 and the power supply unit 7 may be integrally constituted, or may be constituted in a manner separate from each other.

The arithmetic processing unit 5 is composed of a memory circuit 8 for storing correlation data, and a calculating circuit 10 for calculating blood-sugar level by the correlation data stored in the memory circuit 8 and a signal from the light receiving element. The calculating circuit 10 outputs calculation result to the display monitor 6, and outputs it to a computer 11 provided externally of the apparatus through output port (not shown). This computer 11 carries out analysis of measurement data, etc., and outputs analysis result to a printer 12. It is to be noted that the blood-sugar level measuring apparatus 1 may be of a structure including computer 11 and printer 12.

The procedure for measuring blood-sugar level by the blood-sugar level measuring apparatus 1 thus constituted will now be described.

In order to carry out measurement of blood-sugar level, a rubber band 21, etc. is gradually fitted from the front end portion of the finger to allow the finger front end portion to be placed in blood lost state. Blood including hemoglobin absorbs light of wavelength of 400 nm ∼ 630 nm. In the human body tissue placed in blood lost state, there exists tissue fluid including sugar, from which hemoglobin has been removed.

When light of wavelength of, e.g., 400 nm ∼ 630 nm is irradiated from the light emitting element 3 in this state, and is caused to be transmitted through the tissue, absorption/decrease of light related to glucose in the tissue fluid takes place. Thus, the light receiving element 4 receives the absorbed transmitted light to convert it into an electric signal to output the electric signal to the arithmetic processing unit 5.

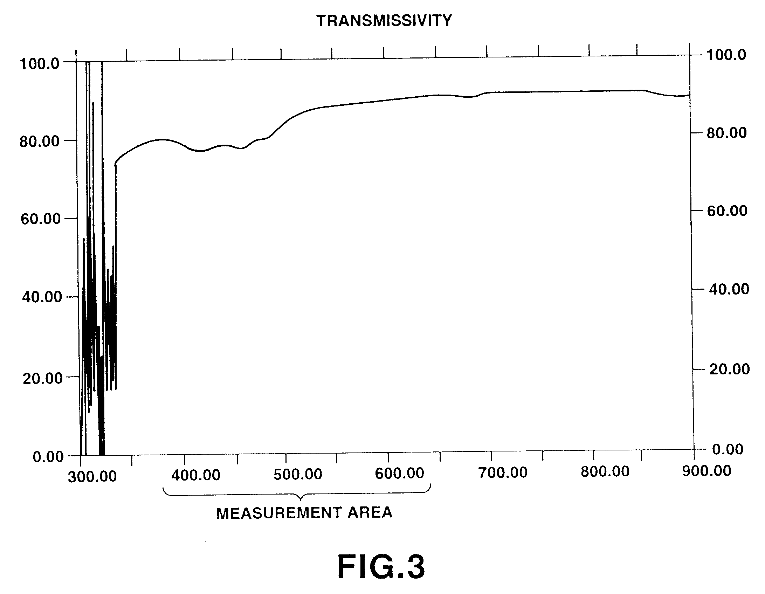

FIG. 3 shows a transmission characteristic, e.g., at blood-sugar level of 100 mg/dl which is output result of the printer 12 of the analysis result of the computer 11. Also as is clear from FIG. 3, a transmitted light has a sufficient transmission characteristic with respect to light of wavelength of 400 nm ∼ 630 nm. Since light in the range of ultraviolet rays less than wavelength 300 nm may give rise to cancer of the skin, etc., it is not suitable as irradiation light.

Because glucose in tissue fluid is related to glucose in blood plasma, it is thus related to blood-sugar level.

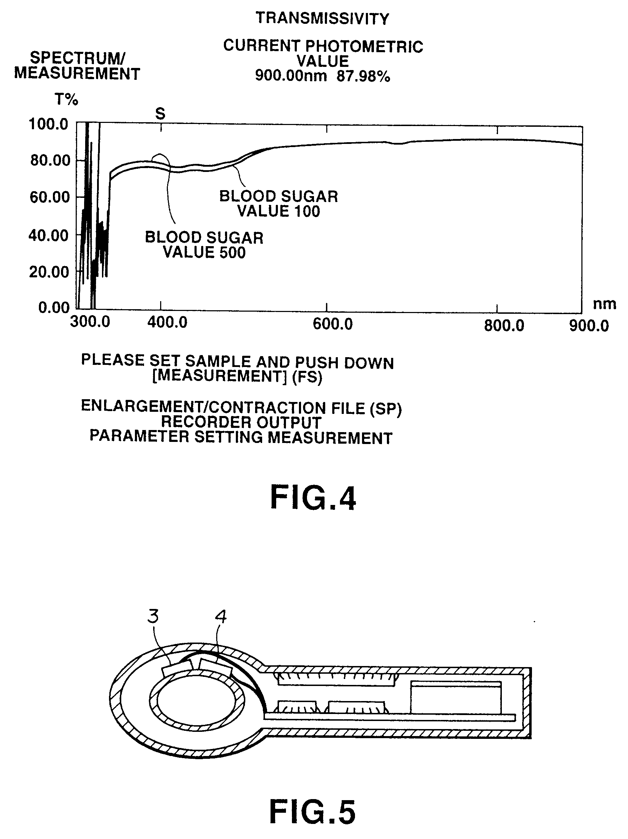

FIG. 4 shows a transmission characteristic of the blood lost tissue by blood-sugar level. Also as is clear from FIG. 4, with respect to transmissivity (transmission factor) of light of wavelength 400 nm ∼ 630 nm, transmissivity of blood-sugar level 500 mg/dl is higher than, e.g., transmissivity of blood-sugar level 100 mg/dl. This is because when hemoglobin is removed so that blood-sugar level becomes higher, bound material in which glucose and blood plasma glycoprotein are bounded increases, so light of wavelength of 400 nm ∼ 630 nm is apt to be transmitted through the tissue.

Namely, an approach is employed to calculate in advance correlation between transmissivity (transmission factor) by glucose in the tissue fluid and blood-sugar level to allow the memory circuit 8 to store it therein as correlation data to detect transmissivity by glucose in the tissue fluid. Thus, calculation of blood-sugar level is carried out.

As stated above, the blood-sugar level measuring apparatus of this embodiment utilizes the fact that the transmission characteristic of light of wavelength 400 nm ∼ 630 nm is improved in the organic tissue in the blood lost state where hemoglobin is removed to measure glucose in the tissue to calculate glucose in blood thus to calculate blood-sugar level. Accordingly, it is possible to safely measure blood-sugar level in a stable manner and in short time.

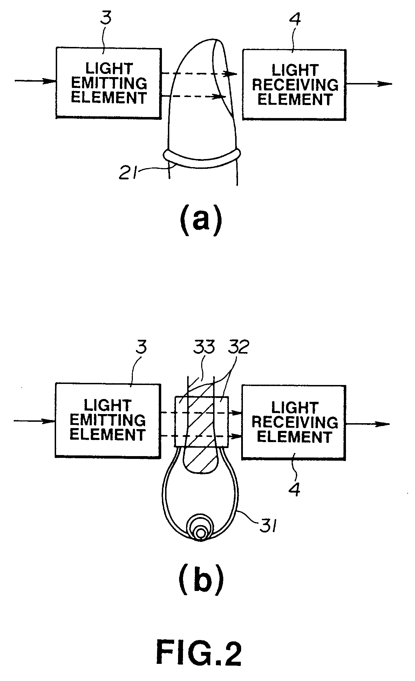

It is to be noted that while approach was employed in this embodiment to allow the front end portion of the finger to be in blood lost state by using rubber band, etc. to measure blood-sugar level, an approach may be employed, in addition to the above, as shown in FIG. 2(b), to hold, e.g., an earlobe 33 by portions opposite to each other of a transparent pad 32 to which pressure is applied toward the inside by a pressure application spring 31 to allow light of wavelength of 400 nm ∼ 630 nm to be transmitted through the earlobe 33 through the transparent pad 32 to measure blood-sugar level. In this case, since the tissue within the earlobe 33 is held by the transparent pad 32 in a pressurized state so that the tissue within the earlobe 33 can be placed in blood lost state, it is possible to easily carry out measurement.

The transparent pad 32 is attached, in addition to the earlobe 33, to the tissue of the portion between fingers, e.g., webbed portion between the thumb and the forefinger, thereby making it possible to realize blood lost state similarly to the measurement at the earlobe. Accordingly, the transparent pad 32 is attached to the webbed portion, thereby making it possible to easily carry out measurement of blood-sugar level similarly to the case where it is attached to the earlobe 33. Namely, any portion of the human body such that portions opposite to each other of the transparent pad 32 to which pressure is applied are attached thereto so that blood lost state can be realized may be adopted for this purpose.

While, in the above-described embodiment, explanation has been given in connection with the case where the light emitting element and the light receiving element are disposed at portions opposite to each other through the blood lost tissue to detect light transmitted through the blood lost tissue, there may be employed, in addition to the above, a configuration adapted to detect reflected light from the blood lost tissue to calculate blood-sugar level. In this case, it is sufficient to employ a configuration as shown in FIG. 5 in which a light emitting element 3 and a light receiving element 4 are disposed in parallel to detect a reflected light from the blood lost tissue by the light receiving element 4.

### Industrial Applicability

As described above, since the blood-sugar level measuring apparatus according to this invention has a configuration to irradiate visible light emitted from the light emitting element into the blood lost tissue to detect a light obtained as the result of the fact that the visible light is transmitted through the blood lost tissue or reflected from the blood lost tissue, it is possible to safely and precisely measure blood-sugar level without causing pain to subject. In addition, it becomes possible to stably measure blood-sugar level in short time by simple configuration.

## Claims

1. A blood-sugar level measuring apparatus comprising:
irradiating means for irradiating visible light into a blood lost tissue caused to be placed in blood lost state of a portion of the organic tissue; and
detecting means for detecting a visible light transmitted through the blood lost tissue or reflected therefrom.

2. A blood-sugar level measuring apparatus as set forth in claim 1, which further comprises:
calculating means for measuring absorptivity of the visible light of the blood lost tissue on the basis of a detection output of the detecting means to calculate a calculated blood-sugar level on the basis of correlation between an absorptivity determined in advance and blood-sugar level and the measured absorptivity.

3. A blood-sugar level measuring apparatus as set forth in claim 2, which further comprises:
display means for displaying calculation result of the calculating means.

4. A blood-sugar level measuring apparatus as set forth in claim 2 or 3,
wherein the calculating means comprises memory means for storing the correlation between the absorptivity determined in advance and the blood-sugar level, and blood-sugar level calculating means for calculating a blood-sugar level, which calculates the calculated blood-sugar value on the basis of the correlation that the memory means stores and the measured absorptivity.

5. A blood-sugar level measuring apparatus as set forth in claim 1, wherein the visible light at least includes a visible light in the range of wavelength of 400 nm ∼ 630 nm.

6. A blood-sugar level measuring apparatus as set forth in claim 1, wherein the blood lost tissue is finger, earlobe or webbed portion between fingers.

7. A blood-sugar level measuring apparatus as set forth in claim 2 or 3, wherein the calculating means comprises output means for outputting predetermined arithmetic processing information.
